# EUROPEAN PATENT SPECIFICATION

(11) **EP 1 241 479 B1**
(45) Date of publication and mention of the grant of the patent: **01.08.2007**
(21) Application number: 01106463.1
(22) Date of filing: 23.03.2001
(51) Int. Cl.: G01N 33/74, G01N 33/543, C07K 14/575, G01N 33/53

(54) **Method of determining urotensin II in body fluids and diagnosis of cardiovascular diseases**
Verfahren zur Bestimmung von Urotensin II in Körperflüssigkeiten und Diagnose von kardiovaskulären Krankheiten
Méthode pour la détermination d'urotensine II dans les fluides corporels et diagnostic des maladies cardiovasculaires

(30) Priority: 12.03.2001 EP 01105418
(43) Date of publication of application: 18.09.2002
(73) Proprietor: Immundiagnostik AG, 64625 Bensheim (DE)
(72) Inventor: Armbruster, Franz Paul, 64625 Bensheim (DE); Stangl, Karl, Humboldt-Universität, 10117 Berlin (DE); Dschietzig, Thomas, Humboldt-Universität, 10117 Berlin (DE); Voelter, Wolfgang, 72070 Tübingen (DE); Heller, Jörg, 64625 Bensheim (DE)
(74) Representative: Benedum, Ulrich Max

(56) References cited:
- WO-A-00/31265
- WO-A-99/35266
- MCMASTER D ET AL: "Characterization of the biologically and antigenically important regions of Urotensin II" WESTERN PHARMACOLOGY SOCIETY. PROCEEDINGS, WESTERN PHARMACOLOGY SOCIETY, TUCSON, AZ, US, vol. 29, 1986, pages 205-208, XP002110992 ISSN: 0083-8969
- CHARTREL N ET AL: "Urotensin II in the central nervous system of the frog Rana ridibunda. Biochemical characterization and immunohistochemical localization." ANNALS OF THE NEW YORK ACADEMY OF SCIENCES. UNITED STATES 15 MAY 1998, vol. 839, 15 May 1998 (1998-05-15), pages 506-507, XP009021153 ISSN: 0077-8923
- CHARTREL NICOLAS ET AL: "Urotensin II in the central nervous system of the frog Rana ridibunda: Immunohistochemical localization and biochemical characterization" JOURNAL OF COMPARATIVE NEUROLOGY, vol. 364, no. 2, 1996, pages 324-339, XP009021138 ISSN: 0021-9967
- SUGO T ET AL: "Identification of urotensin II-related peptide as the urotensin II-immunoreactive molecule in the rat brain" BIOCHEMICAL AND BIOPHYSICAL RESEARCH COMMUNICATIONS, ACADEMIC PRESS INC. ORLANDO, FL, US, vol. 310, no. 3, 24 October 2003 (2003-10-24), pages 860-868, XP004461174 ISSN: 0006-291X

## Description

The invention relates to methods of determining urotensin II in body fluids and diagnosis of cardiovascular and vascular diseases.

Urotensin II (U II) is a somatostatin like-peptide initially isolated from the caudal neurosecretory system of teleost fish [Pearson D et al. (1980) PNAS U.S.A., 77(8), 5021-4]. The cDNA of the urotensin II precursor has been cloned from a number of species, including human, mouse, rat and frog [WO 99/35266 (PCT/US99/00489); US 6,075,137; Coulouarn Y et al. (1998), PNAS U.S.A., 95(26), 15803-8; Coulouarn Y et al. (1999), FEBS Lett., 457, 28-32; Ames RS et al. (1999), Nature 401, 282-6]. The human urotensin II precursor consists of 124 amino acid residues which is proteolytically cleaved to generate a bioactive peptide of 11 residues (Swiss-Prot 095399; Conlon JM et al. (1990) FEBS Lett, 266(1-2), 37-40]). A region of 6 residues (CFWKYC) is cyclisized by disulfide bridge formation and responsible for the biological activity of urotensin II (Coulouarn Y et al. (1998), PNAS U.S.A., 95(26), 15803-8; Itoh H et al. (1988), Eur. J. Pharmacol., 149, 61-6). This sequence is evolutionarily conserved from fish to human and shows high homology to somatostatin.

Human urotensin II precursor mRNA is actively expressed in many tissues of the central nervous system and the periphery, however expression in the spinal cord and medulla oblongata is by far higher than in any other tissue. urotensin II binds with high affinity to GPR14, a protein G-coupled orphan receptor with similarity to members of the somatostatin/opioid receptor family (Ames RS et al. (1999) Nature 401, 282-6; Nothacker HP et al. (1999), Nat. Cell Biol., 1, 383-385; Liu Q et al. (1999), Biochem. Biophys. Res. Commun., 266, 174-8; Mori M et al. (1999), Biochem. Biophys. Res. Commun., 265, 123-9). GPR14 is widely expressed in human tissues, including the left atrium and ventricle of the heart as well as in smooth muscle cells from the coronary artery and aorta. Whether human urotensin II is released locally or circulates in the plasma some distance from the target receptors remains to be elucidated.

Urotensin II exhibits various physiological and pharmacological effects, including general smooth muscle contracting activity [Davenport AP et al. (2000), TiPS 21, 80-82]. Both potency and efficacy of urotensin II induced vasoconstriction as measured *in vitro* is an order of magnitude higher than that of other peptides like endothelin-1, noradrenaline, serotonin and angiotensin II [Ames RS et al. (1999) Nature 401, 282-6; MacLean MR et al. (2000) Br. J. Pharmacol., 130, 201-204]. Systemic *in vivo*-administration of human urotensin II to anaesthetized monkeys caused marked systemic vasoconstriction, and resulted in severe myocardial contractile dysfunction and fatal circulatory collapse [Ames RS et al. (1999), Nature 401, 282-6]. In rat, the vasoconstrictive activity of human urotensin II lacks systemic pressure activity and is limited to the isolated thoracic aorta [Katano Y et al. (2000), Eur. J. Pharmacol., 402, 209-211; Bottrill FE et al. (2000) Br. J. Pharmacol., 130, 1865-1870]. Thus, the urotensin II peptide exhibits significant anatomical and functional differences in various species. urotensin II plasma values in humans as well as its potential role in human heart failure (CHF), however, have not yet been investigated.

McMaster et al. disclose in Proc. West. Pharmacol. Soc., 29:205-208 (1986) a radioimmunoassay for urotensin II using an antiserum raised in rabbits against Gillichthys U II and wherein the antibody recognition site in this antiserum is directed to the conformation imparted by the intact disulfide ring region of the U II peptide, the biologically and antigenically important region of U II.

One disadvantage of prior art methods for determining human urotensin II in body fluids is that antibodies raised against peptides from urotensin II tend to bind human proteins other than urotensin II.

Another disadvantage of prior art methods for determining urotensin II in plasma is that the measured levels of urotensin II do not relate to the known physiological effects of urotensin II and that the methods per se are not based on a biological model.

It was therefore the object of the invention to develop a both sensitive and specific immunoassay for the quantifica-tion of urotensin II in serum and plasma which can be applied to monitor the biological activity of this peptide.

This object has been achieved by a method as claimed in claim 1. Further embodiments of the invention are described in dependent claims 2 to 6.

In an embodiment of the invention the method of determining the biological active urotensin II in a sample of a body fluid, comprises contacting the sample with polyclonal and/or monoclonal antibodies which bind to a urotensin II structure having the sequence
CFWKYCX SEQ ID NO: 1
wherein C is cystein, F phenylalanine, W tryptophane, K lysine, Y tyrosine, wherein X is optional and either β-alanine, valine, or isoleucine and the region of CFWKYC is cyclisized by a disulfide bridge formation.

The antibodies are elicted by immunisation with a cyclic peptide having the sequence
ETPDCFWKYC-βAla-C, SEQ ID NO: 2
with a disulfide bridge formed between Cys⁵ and Cys¹⁰, and comprising the artificial sequence βAla-Cys, optionally conjugated with a carrier peptide.

In another method of the invention, the cyclic urotensin II peptide is bound to a stationary phase and the antibodies against urotensin II-β-Ala-Cys are purified by their specific binding to cyclic human urotensin II.

The method for determining purotensin II is preferably a competitive binding assay such as ELISA, RIA or EIA.

Method as claimed is being used in another embodiment of the invention for the diagnosis of cardiovascular diseases, heptarenal syndrome, liver cirrhosis, diseases involving haemodynamic alterations associated with portal hypertension, pathological conditions involving abnormal levels of vasocontrictive substances or for diagnosis of neurodegenerative diseases or traumatism of the spinal cord.

The application further teaches to a method of preparing cyclic urotensin II peptides comprising the step of cyclic oxidation of cysteins 5 and 10 following the synthesis of the primary urotensin II structure.

A detailed description of exemplary embodiments of the invention is provided below with reference to the following drawings, in which:
- Fig. 1A: is a chromatogramm of the synthetic cyclic urotensin II peptide by analytical HPLC: Stationary phase: Nucleosil 300 5µ C₁₈, eluent: A: 0.35% TFA/H₂O; B: 0.29% TFA/60% CH₃CN; gradient: 10-90% B in 36 min, λ=214 nm, 1 ml/min;
- Fig. 1B: is a matrix-assisted-laser-desorption-mass-spectrum of the synthetic cyclic urotensin II of the invention: Nₒ of shots: 76, matrix: α-cyno-4-hydroxycinnamic acid;
- Fig. 1C: is a chromatogramm of the synthetic (cyclic urotensin II) -βAla-Cys peptide by analytical HPLC: Stationary phase: Nucleosil 300 5µ C₁₈, eluent: A: 0.35% TFA/H₂O;B: 0.29% TFA/60% CH₃CN; gradient: 10-90% B in 36 min, λ=214 nm, 1 ml/min;
- Fig. 1D: is a matrix-assisted-laser-desorption-mass-spectrum of the synthetic (cyclic urotensin II)-βAla-Cys which was used for immunisation: Nₒ of shots: 87, matrix: α-cyno-4-hydroxycinnamic acid;
- Fig. 2: is a diagramm of calibration curves with different incubation schemes.
--◆- 3 hours simultaneously standard and antibody
-- -- sequentially 1 hour standard, 2 hours antibody
--x-- sequentially 1 hour standards, 1 hour antibody
- Fig. 3: is a diagramm showing the recovery of synthetic cyclic urotensin II peptide added to 10 different plasma samples;
- Fig. 4: is a diagramm showing the linearity of the U-II ELISA of the invention (Thirteen samples were assayed in a dilution of 1:10 and 1:30 in assay buffer) ;
- Fig. 5: comparative analysis of 14 plasma samples; human urotensin II has been determined by the method of the invention and a commercial human urotensin II RIA assay (Phoenix Pharmaceuticals Inc.) with antibodies against linear human urotensin II. Weak correlation is seen.

### EXAMPLE

### Example 1 - Synthesis of urotensin II Cyclic urotensin II peptide with the sequence

ETPDCFWKYC (SEQ ID NO: 1)
   (with a disulfide bridge formed between Cys⁵ and Cys¹⁰)
was synthesized in a stepwise manner using Fmoc-technology with an automated ECOSYN P peptide synthesizer (Eppendorf-Biotronik, Hamburg, Germany).

Starting with 1g (0.2 mMol) Fmoc-Cys (Trt) Trt-S-PHB-Tentagel-resin (Rapp Polymere, Tübingen, Germany), each Fmoc-amino acid derivative (0.8 mMol) was coupled to the resin using 0.26g (0.8 mMol) TBTU (2-(1H-benzotriazole-1-yl)-1,1.3,3-tetramethyluronium-tetrafluorborate) and 1.1 ml DIEA-solution (20% diisopropylethylamine in dimethylformamide). The activation time was 4 min, the coupling time 30 min. The following protecting groups were used: cysteine: Trityl; lysine and tryptophane: t-butyloxycarbonyl; tyrosine and threonine: t-butyl; aspartic- and glutamic acid: O-tert-butylester. After introduction of the last amino acid, the Fmoc-group was removed using 25% piperidine in dimethylformamide (20 min) and the Boc-group was introduced using Boc₂O/DIEA in dichlormethane (10 fold excess; 1h, room temperature). The fully protected peptide was removed from the resin using a mixture of acetic acid, trifluorethanol, dichlormethane (40/10/50 ml) by stirring for 1h at room temperature. After removal of the solvents, the peptide was precipitated from dichlormethane with petrolether yielding 340 mg. Oxidation of the peptide (0.16 mMol) was achieved using 0.3 g iodine dissolved in hexafluor-2-propanol/dichlormethane (5/35 ml). The substrate in 10 ml dichlormethane and 5 ml hexafluor-2-propanol was added dropwise to the iodine solution. After 9 min, the reaction was stopped by addition of an ice-cold solution of ascorbinic acid (0.92g) and 0.7g ammonium acetate. After separation of the organic layer, the water phase was extracted twice with chloroform. The combined organic phases were dried with sodium sulfate. The final deprotection of the remaining protecting groups was performed using a mixture of trifluoracetic acid/thioanisole/anisole/triisopropylsilane (12/0.6/0.3/0.1 ml) for 2 h at room temperature. After filtration from the resin, the peptide was precipitated with diethylether. The precipitated crude peptide was redissolved in 20% acetic acid and subsequently lyophilized. The yield was 240 mg. Finally, the peptide was purified by semipreparative HPLC using a Nucleosil 300 5µ C_{1B}-column (Macherey-Nagel, Düren, Germany). The gradient was from 10-90% B in 36 min (A: 0.35% TFA/H₂O; B: 80% CH₃CN, 0.29% TFA/H₂O). Flowrate was 3.5 ml/min, detection at λ214 nm.

For coupling of the urotensin II to bTG, the peptide with the following sequence was used
ETPDCFWKYC-βAla-C SEQ ID NO: 2
(with a disulfide bridge formed between Cys⁵ and Cys¹⁰). The synthesis of this peptide was performed in a similar manner (0.2 mMol scale) as for the former one except that for the protection of Cys⁵ and Cys¹⁰, the Mmt (Methoxytrityl)-group was used. Following synthesis, the peptide and the Mmt-groups were removed simultaneously from the resin. The disulfide bridge was formed by oxidation with a mixture of dimethylsulfoxide/dimethylformamide/water (30/15/5; 100 ml) over night at room temperature. After removal of the solvents, the peptide was precipitated from the dichlormethane by adding petrolether. Removal of the remaining protection groups and HPLC purification was done as described above.

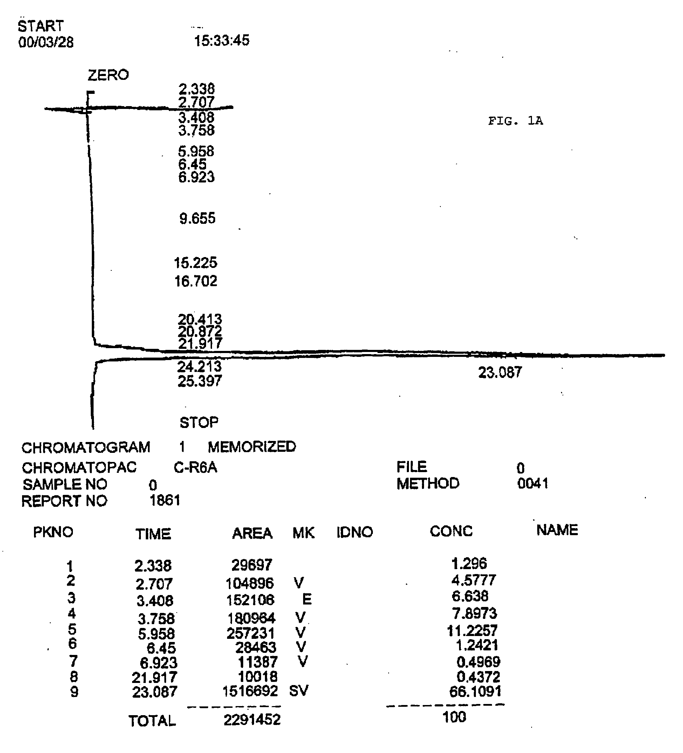

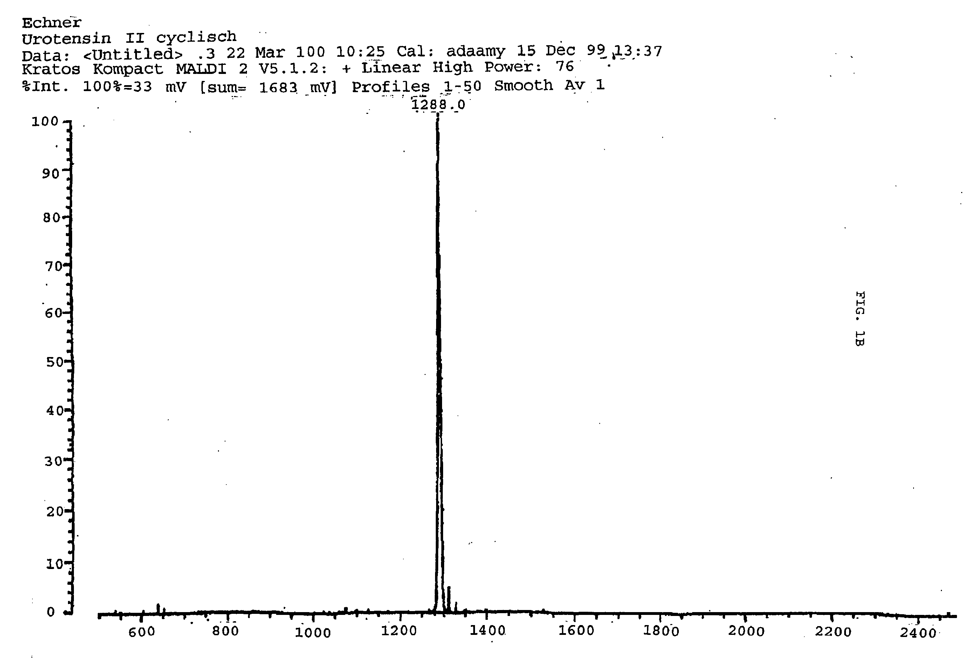

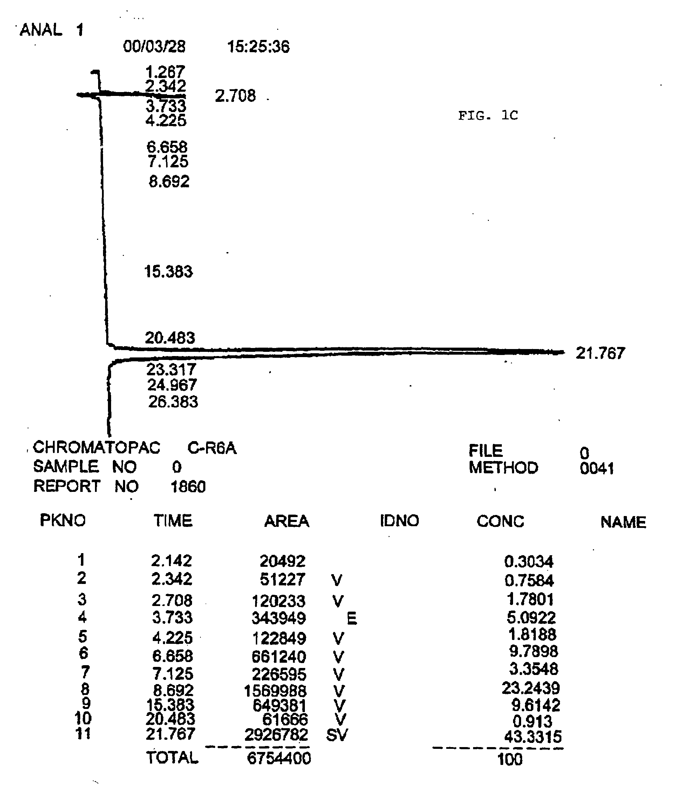

Both peptides were fully analyzed and characterized by analytical HPLC and mass-spectometry (see Figs. 1A-D).

### Example 2 - Immunisation

The cyclic urotensin II peptide coupled to beta-thromboglobulin (bTG) was emulsified in ABM^{®} (Lenaris GmbH, Wertheim, Germany) and used to immunize four white Zealand rabbits. Test bleeds were taken one week after boosts and rabbits were boostered three times in an eight week period.

The immunological response was monitored by an ELISA with urotensin II peptides immobilized on microtiter plates. Addition of serial dilutions of the rabbit antisera was followed by horseradish peroxidase (HRPO) labelled donkey anti rabbit Ig (DAKO, Germany). To visualize the immunoreactivity, the ready to use single component TMB substrate was employed (BioFX, USA). The bleed obtained after the third boost was used for further development of the assay.

### Example 3 - Antibody Purification

urotensin II specific antibodies were purified from antiserum using immunoaffinity chromatography on HiTrap minicolumns (Pharmacia, Sweden) . According to the manufacturer's protocol, 0.3 g of synthetic cyclic urotensin II peptide was bound to the resin. 5 ml of antiserum were filtered through a 0.45 µm Millex-Filter (Millipore, USA) diluted with 2 volumes of 50 mM borate buffer pH 7.0 and loaded at room temperature onto the HiTrap minicolumn at a flow rate of 0.5 ml/min. After washing the resin, specific antibodies were eluted with 0.1 M citrate buffer pH 1.7 at a flow rate of 1 ml/min. Elution was monitored by UV detection at 280 nm, and fractions of 0.5 ml were collected onto 0.5 ml of 0.5 M borate buffer pH 10 to ensure immediate neutralisation. Concentration of the specific Ig was determined with a modified *µ*BCA protein assay (Pierce, The Netherlands).

### Example 4 - Assay Development

Microtiter plates (Nunc Maxisorp High Binding, NUNC, Denmark) were coated for 3 h at room temperature with 0.1 ml of 0.05M borate buffer, pH 9.6, containing 250 pg of synthetic cyclic urotensin II peptide. Remaining binding sites were blocked with 0.35 ml of 0.05 M borate buffer, pH 9.6, containing 2% bovine serum albumine (Sigma, USA) and 5% glucose (Merck, Germany). Plates were then dried over night at 30°C and finally sealed in foil pouches with desicant and stored at 4°C until use.

Variations of the assay parameters (concentration of reagents, temperature, time of incubation, pH of buffer) were tested during the development to optimise sensitivity, recovery and precision (see Results).

The following protocol was selected for further measurements: Plasma samples and antibodies were diluted in 50mM Tris/HCl, pH 7.0, containing 0.1% BSA, and 0.1% Triton X-100. 0.05 ml of diluted sample was incubated for 1 h at 37°C together with 0.05 ml of diluted antibody (final dilution of 1:4000). Then, 0.1 ml of the mixture was transferred to microtiter wells coated with synthetic urotensin II peptides, and incubated for 2 hours at 37°C. A washing step was followed by incubation with conjugate (donkey anti rabbit IgG coupled to horseradish peroxidase, DAKO, Germany) diluted 1:10000 in stabilizing buffer (Orgenteg, Germany) for 1 h at 37°C on a shaker. Following a final washing step, 0.1 ml tetramethylbenzidin (TMB, BioFX, USA) was added, and incubation proceeded for 20 min at room temperature. Reaction was stopped by adding 0.05 ml of 1N H₂SO₄. Absorption was measured in a standard microtiter plate photometer at 450 nm with 620 nm as a reference.

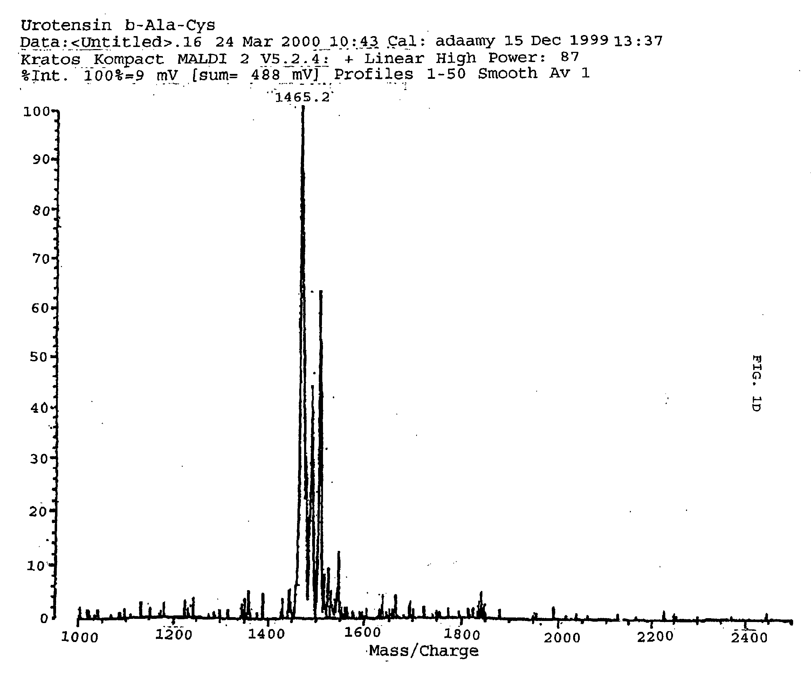

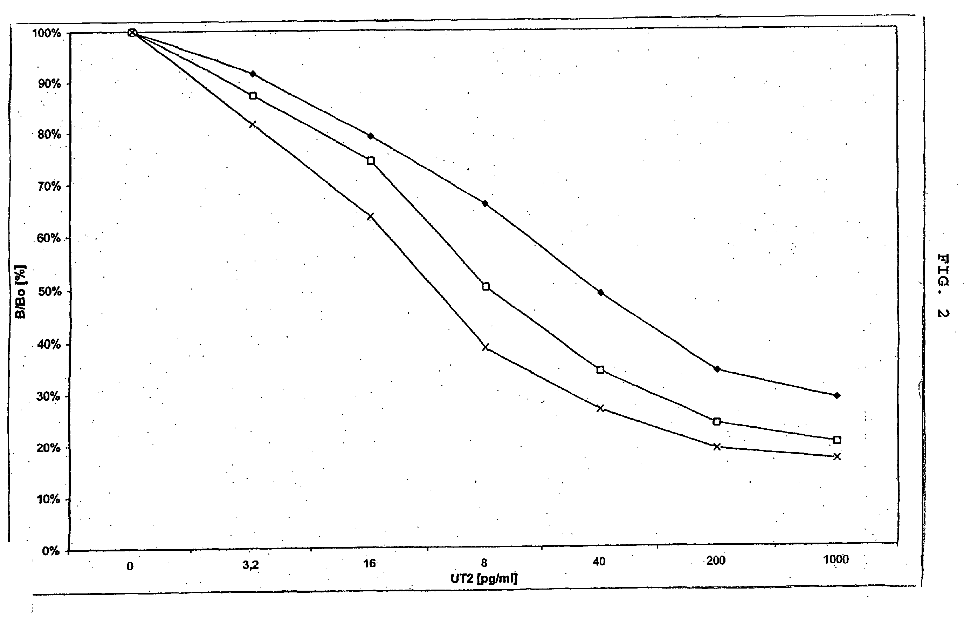

Recovery and linearity were studied in plasma samples partially spiked with synthetic urotensin II peptides. A typical calibration curve is shown in Fig. 2. The detection limit is defined as the concentration for a calculated displacement of 5%, i.e. at 95% B/Bₒ. The calculated detection limit was calculated to be 3 pg/ml. Values are given as mean ± SD. The statistical methods used include linear regression and linear correlation coefficient.

Results and Discussion. After the third boost, serum obtained from the rabbit immunized with bTG-conjugated antigen showed significant binding of >0.5 OD in the screening assay at dilutions greater than 1:5000. For further assay development, urotensin II specific antibodies were isolated by affinity purification on synthetic cyclic urotensin II peptides.

Efficiency of coating microtiter plates with the peptide antigen strongly depend on the brand and batch of plates. Best results were obtained with Nunc Maxisorp High binding plates of batch #048848. Sequential incubation of samples and antibodies significantly increased the sensitivity of the immunoassay. Best results were obtained at 1h and 2 h assay setup, where the ratio of absolute signal of Bₒ to sensitivity was optimal. Typical calibration curves obtained are shown in Fig 2. The detection limit was calculated to be 3 pg/ml (95% B/Bₒ).

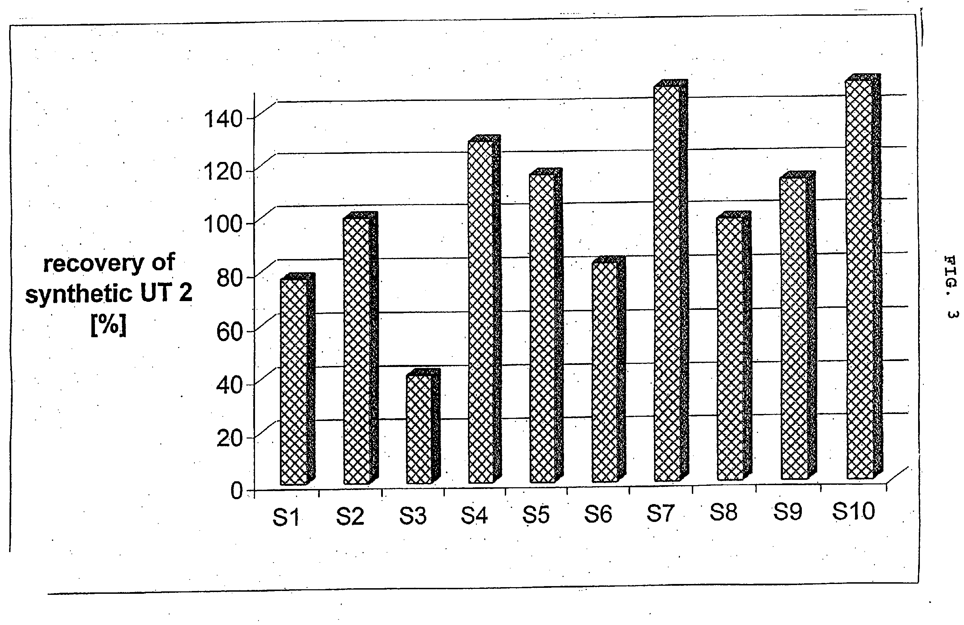

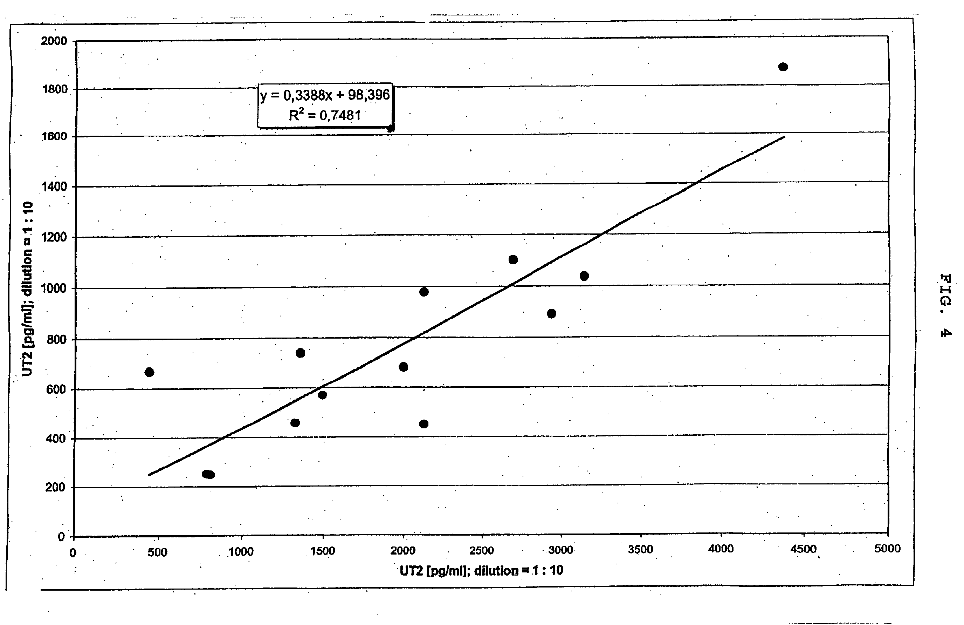

Similar displacement curves and recovery were found in serum and plasma samples after dilution of 1:10 and higher. At dilutions of less than 1:10, matrix effects cause interference and a high background to signal ratio. Ten samples were spiked with 100 pg/ml urotensin II peptide for recovery validation. Mean recovery was 94%, nevertheless a high variation of 35% was observed (Fig 3). Thirteen samples were assayed in a dilution of 1:10 and 1:30. Results given in Fig. 4 show good linearity in the range of 3-250 pg/ml (correlation coefficient r=0.87).

Crossreactivity with human endothelin-1, atrial and brain natriuretic peptide, urodilatin, adrenomedullin, and relaxin was <0.1%.

**TABLE 1**

| Cross-reacitvity with various vasoactive peptides | | |
|---|---|---|
| vasoactive peptide | tested till | cross-reactivity [%] |
| human Endothelin 1-21 | 0.1 µg/ml | <0.01 |
| human big Endothelin 1-38 | 0.1 µg/ml | <0.01 |
| human Urodilatin | 10 µg/ml | <0.01 |
| human ANP 1-28 | 10 µg/ml | 0.05 |
| human BNP 32 | 10 µg/ml | <0.01 |
| Adrenomedullin 1-52 | 10 µg/ml | 0.1 |
| Relaxin | 2 µg/ml | 0.2 |
| Somatostatin | 10 µg/ml | < 0.01 |

In summary, the assay according to the invention is based on (cyclic urotensin II) -affinity purified antibodies raised against a synthetic cyclic-urotensin II-βAla-Cys peptide. Using cyclic urotensin II peptides immobilized on microtiter plates, the obtained competitive ELISA showed linearity in the range of 3 to 250 pg/ml, a lower detection limit of 3 pg/ml (95% B/Bo) and a mean recovery of 94%. Interassay variation was lower than 15%. Crossreactivity with human endothelin-1, atrial and brain natriuretic peptide, urodilatin, adrenomedullin, and relaxin was < 0.1%. The immunoassay according to the invention therefore provides a tool for the direct quantification of the urotensin II level in human plasma, and thus contributes to differential diagnosis of cardiovascular and neurodegenerative diseases.

Contrary to prior art methods, synthethic cyclic U-II peptides linked to βAla-Cys have been used as antigen in the immunisation protocol to elicit antibodies against natural cyclic urotensin II which has been used on the other hand for affinity-purification of the elicited antibodies. Consequently, the obtained antibodies were not merely specific for the urotensin II sequence but highly-specific for the cyclic structure of urotensin II which is most conserved thoughout species, binds to the corresponding receptor and comprises the biological activity. Immunoassays on basis of antibodies against the primary sequences of urotensin II as suggested in WO 99/35266 or WO 00/31265 or Phoenix™-Assay would not detect the biological active urotensin II.

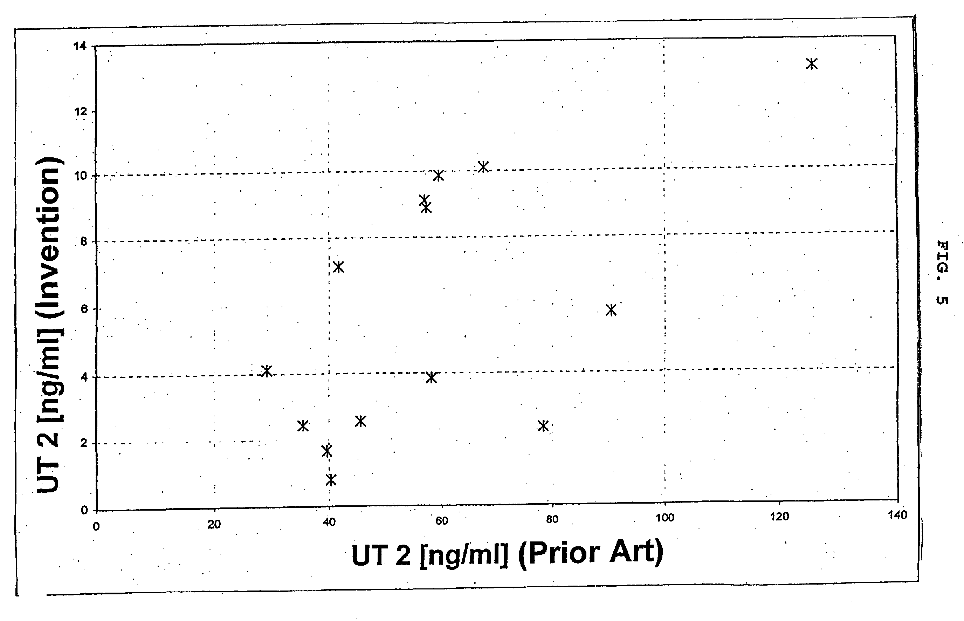

Fig. 5 shows a comparative analysis of a commercial urotensin II RIA assay (Phoenix Pharmaceutical Inc., Belmont, California) which detects the linear urotensin II structure and the afore-mentioned assay of the invention. Fourteen plasma samples were measured. As shown in Fig. 5 remarably higher values were found in the assay of the state of the art which suggests that the prior art assay detects urotensin II proteins and structures which have no biological activity. Given the strong vasoconstrictive activity reported for urotensin II it seems reasonable to assume that the concentration of this peptide is lower compared to other vasoactive peptides.

### Example 5 - Determination of plasma urotensin II in patients with cardiovascular diseases

### Patients and protocol

### Group 1: severe CHF

Eleven patients - six suffering from CHF secondary to ischemic heart disease (IHD), and five demonstrating dilated cardiomyopathy (DCM) - were enrolled (4 females, 7 males; age, 55±5 years; left ventricular ejection fraction, 18±3%.) Inclusion criteria were i) left heart decompensation (orthopnea, signs of severe pulmonary congestion) not due to acute ischemia, and ii) pulmonary capillary wedge pressure >25 mmHg, and cardiac index < 2.5L/min/m².

### Group 2: moderate CHF

Patients undergoing haemodynamic evaluation prior to partial ventriculectomy were assigned to this group (n=10, 4 females, 6 males; age, 54±7 years; ejection fraction, 29±5%; 5 IHD and 5DCM patients) if they were i) in NYHA class II and if they displayed ii) a pulmonary capillary wedge pressure < 20 mmHg and cardiac index > 2.5 L/min/m².

### Group 3: controls

Thirteen individuals (4 females, 9 males; age 55±4 years; ejection fraction, 65±5%) in whom cardiac catheterization revealed no structural cardiovascular disease were enrolled.

### Protocol, instrumentation, and blood sampling

Protocol, instrumentation, and blood sampling were described previously. (Stangl K. et al. (2000) Circulation 102, 1132-8.) In brief, CHF patients were treated over 24 h with the vasodilator sodium nitroprusside (SNP) to achieve a systemic vascular resistance of 800-1000 dyn/s/cm⁻⁵ without lowering mean arterial blood pressure below 60 mm Hg. All CHF patients were on stable oral therapy: all on ACE inhibitors and diuretics; seventeen on nitrates; thirteen on β-blockers; and nine on digitalis.

Catheters were positioned in the pulmonary artery (PA), coronary sinus (CS), left ventricle (LV), radial artery (A), and antecubital vein (V). Blood sampling and haemodynamic measurements were performed at baseline and at 2, 12, 18 and 24 h after initiation of therapy. In groups 1 and 2, the left ventricular catheter and the coronary sinus catheter were removed for safety reasons after baseline measurement and after 4 h, respectively. Baseline levels of urotensin II did not differ between LV and A in all groups, which proved the reliability of concentrations detected in A for monitoring the subsequent treatment throughout 24 h.

This study was approved by the local Ethics Commitee. Prior to the study, all patients gave their informed, written consent. The study was performed in accordance with the Helsinki Declaration of 1975, as revised in 1983.

### Urotensin II gene expression in myocardium and vasculature Left ventricles

Control samples were human total left ventricular RNA from healthy individuals (n=4) (Invitrogen) and sample from one donor heart that was not transplanted. Failing myocardium was from patients with end-stage CHF (LV ejection fraction, 17±3%) who demonstrated DCM (n=3) or IHD (n=3) and underwent partial ventriculectomy or transplantation.

### Right atria and vessels

Samples from right atrium (n=8), mammary artery (n=6), and saphenous vein (n=6) were obtained from patients with normal heart function; vascular specimens (n=4 each) were also taken from patients with CHF (n=4; LV ejection fraction, 20±3%). All these patients underwent bypass surgery. We furthermore used samples from dilated right atria of DCM patients (n=4; LV ejection fraction, 18±2%) who underwent partial ventruculectomy. In all patients, heart function had been assessed by preoperative catheterization.

### Analysis of mRNA

We isolated total RNA and performed reverse transcription according to standard procedures. PCR amplification of single-stranded cDNA was then performed utilizing primer for prepro urotensin II as described in Coulouarn Y et al. (1998), Proc. Natl. Acad. Sci. USA., 95, 15803-8 (TIB MOLBIOL), and for human glycerolaldehyde-3-phosphate dehydrogenase (GAPDH)
SEQ ID NO: 3
   upstream: 5' TGA AGG TCG GAG TCA ACG GAT TTG GT 3'
SEQ ID NO: 4
   downstream: 5' CAT GTG GGC CAT GAG GTC CAC CAC 3'
   (Clontech).
   Southern blot hybridization was conducted for quantization of the amplified sequences using radioactively labeled specific oligo prepro U-II:
SEQ ID NO: 5
   5' CCA TAC AAG AAA CGT GAG ACT CCT GAT TGC 3'
Subsequent to autoradiography, data were normalized to GAPDH mRNA expression.

### Statistics

Data are presented as mean±SEM unless otherwise indicated. An error probability of P<0.05 was regarded as significant. Baseline values for unpaired data (haemodynamic parameters, mRNA data) were compared using the Kruskal-Wallis ANOVA or the Mann-Whitney rank sum test. Baseline values paired data (peptide concentrations at different sites of measurement) were compared using the Friedmann ANOVA on ranks. Differences between groups over time (haemodynamics and peptide levels) were analyzed with a nonparametric ANOVA for repeated measures. In each case, a multiple-comparison procedure with Bonferroni-Holm adjustment of P was carried out after global testing. (Itoh H et al. (1988), Eur. J. Pharmacol., 149, 61-6.)

### Results

### Haemodynamics

Table 2 illustrates the significant deterioration of baseline haemodynamics with the progression of CHF. In severe CHF, SNP treatment (Table 2) induced significant haemodynamic improvement: i.e., decreases in pulmonary arterial pressure (-41±4%), pulmonary capillary wedge pressure (-55±3%), systemic (-63±5%) and pulmonary vascular resistance (-60±3%), as well as an increase in cardiac index (-78±3%). Maximum effects were already achieved within the first 2 h of treatment. In moderate CHF, only the decreases in mean arterial pressure (-32±2%) and systemic vascular resistance (-53±3%) were significant.

**TABLE 2**

| Haemodynamics at Baseline and During Vasodilator Therapy | | | | | | | |
|---|---|---|---|---|---|---|---|
| **Time(h)** | **HR** | **MAP** | **MPAP** | **PCWP** | **Cl** | **SVR** | **PVR** |
| **Severe CHF** | | | | | | | |
| 0 | 91 ± 4 | 83 ± 2 | 46 ± 2^{b} | 33 ± 2^{b} | 1.8 ± 0.2^{b} | 1921 ± 137 | 356 ± 39^{b} |
| 24 | 86 ± 3 | 61 ± 3^{d} | 27 ± 2^{d} | 15 ± 1^{d} | 3.2 ± 0.1^{d} | 720 ± 21^{d} | 144 ± 15^{d} |

| **Moderate CHF** | | | | | | | |
|---|---|---|---|---|---|---|---|
| 0 | 84 ± 4 | 87 ± 5 | 22 ± 2 | 14 ± 2 | 2.9 ± 0.3 | 1602 ± 35^{c} | 160 ± 13^{c} |
| 24 | 77 ± 8 | 60 ± 2^{d} | 19 ± 2 | 11 ± 2 | 3.6 ± 0.7 | 757 ± 72^{d} | 106 ± 46 |
| **Controls** | | | | | | | |
| 0 | 80 ± 3 | 92 ± 2^{a} | 14 ± 1^{a} | 10 ± 1^{a} | 3.5 ± 0.1^{a} | 707 ± 26^{a} | 92 ± 48^{a} |

| | | | | | | | |
|---|---|---|---|---|---|---|---|
| HR, Heart rate; MAP, mean arterial pressure (mm Hg); MPAP, mean pulmonary arterial pressure; PCWP, pulmonary capillary wedge pressure; CI, cardiac index (L/min/m²); SVR/PVR, systemic /pulmonary vascular resistance (dyn/s/cm⁻⁵). P<.05; ^{a}, severe CHF vs. controls; ^{b}, severe vs. moderate CHF; ^{c}, moderate CHF vs. controls; ^{d}, vs. baseline. | | | | | | | |

### Plasma Urotensin II

Depending on the sites of measurement, baseline levels in controls ranged between 8.4±2.4 and 1.11±4.0 ng/mL (V). In moderate CHF, values were measured between 8.5±3.0 (LV) and 10.5±2.2 ng/mL (V); and in patients with severe CHF, we determined levels between 11.0±4.0 (CS) and 13.0±3.2 ng/mL (PA). We selected significant differences neither between the 3 groups nor between sites of measurement within a single group of patients.

During vasodilator therapy, the significant haemodynamic improvement achieved in the patients with severe CHF did not result in changes of circulating urotensin II over 24 h (Fig. 2b). Patients with moderate CHF similarly exhibits no alteration of plasma urotensin II during treatment (data not shown).

### Urotensin II mRNA in myocardium and vasculature

The expression of prepro urotensin II mRNA in tissue samples obtained from patients with preserved heart function and patients with end-stage CHF. We detected different gene expression neither in right atrial (control, 1.0±0.2 vs. CHF, 1.3±0.2 arbitrary units) nor in left ventricular myocardium (1.0±0.3 vs. 0.7±0.3 units) . Similarly, prepro urotensin II mRNA levels in mammary arteries and saphenous veins from controls were comparable to those detected in end-stage CHF (mammary artery, 1.0±0.4 vs. 1.3±0.4 units; saphenous vein, 1.0±0.3 vs. 0.8±0.2 units).

### Discussion

Owing to its potent vasoconstrictor effects, urotensin II represents a candidate regulator of vascular tone in physiological and pathophysiological states. Plasma urotensin II seems independent of the severity of CHF and shows no response to haemodynamic improvement in severe CHF. Significant differences between sites of measurement - indicating net release or consumption across pulmonary circulation (LV minus PA levels), coronary (CS-LV), or peripheral vascular beds (V-A) - were found in none of the groups. The gene expression of urotensin II in myocardial and vascular samples from end-stage CHF was comparable to that determined in control specimens.

Until now, no studies have been carried out to investigate the role of urotensin II in CHF. Judging from our findings, involvement of urotensin II in the pathophysiology of human CHF cannot be ruled out. First, because no relevant spare receptor reserve seams to exist in the urotensin II system (Itoh H et al. (1988), Eur. J. Pharmacol., 149, 61-6.) and because urotensin II behaves as a "pseudo-irreversible" ligand like endothelin-1 (Douglas SA et al. (2000), Br. J. Pharmacol., 131, 1262-1274). Even modest changes in the expression of urotension II receptors may have profound effects on contractile efficacy and, accordingly, on pathophysiological impact. The new test system for cyclic urotensin II according to the invention will serve to clarify these items. Second, urotensin II exhibits a unique "anatomically restricted" contractile profile: In rats and dogs, it is a selective aortocoronary constrictor (Douglas SA et al. (2000), Br. J. Pharmacol., 131, 1262-1274) although it has a broader spectrum of target vessels in primates and humans (Maguire JJ et al. (2000), Br. J. Pharmacol., 131, 441-6). However, human mammary arteries and saphenous veins are highly sensitive to urotensin II which proves the existence of a functional urotensin II system in the vessels utilized in our study. It appears therefore unlikely that a site-restricted vascular regulation in CHF remains in the type of vessels examined.

### Example 6 - Determination of plasma urotensin II levels in patients with cirrhosis

### Patients and Methods

Patients with portal hypertension are characterized by splanchnic vasodilation and activation of endogenous vasoconstrictor. Since urotensin II is the strongest endogenous vasoconstrictor of large arterial vessels discovered so far [Ames RS et al. (1999), Nature 401, 282-6] and in contrast relaxes rat mesenteric resistance vessels (Botrill FE et al. (2000), Br. J. Pharmacol., 130, 1865-70) we determined plasma levels in patients with cirrhosis and healthy control persons.

Plasma urotensin II levels were measured by ELISA in accordance with the invention in 20 patients with cirrhosis and portal hypertension (wedged hepatic venous pressure gradient =19±1 mmHg) and in 10 aged matched healthy controls. Additionally, plasma urotensin II levels were compared between hepatic venous and portal venous blood of 10 patients with cirrhosis prior to TIPS insertion.

### Results

urotensin II plasma levels were significantly higher in cirrhotic patients (10640±1751 pg/ml) compared to control persons (3921±889 pg/ml, p< 0.005). In cirrhotic patients undergoing TIPS implantation hepatic venous urotensin II plasma levels were significantly higher (14756±3500 pg/ml) compared to portal venous plasma levels (11931±2973 pg/ml, p< 0.02).

In conclusion, urotensin II plasma levels are increased in patients with cirrhosis. The gradient between portal and hepatic venous urotensin II levels probably indicates the hepatic origin of urotensin II in cirrhosis. Thus, the plasma level of urotensin II plays a role in heamodynamic alterations associated with portal hypertension.

Plasma urotensin II levels were also measured in patients having a liver stent. Blood samples were taken from V. portae (entry) and V. cava inferior (exit) of the liver and measured and it was found that the levels of urotensin II in blood was increased after passage of the liver up to 30fold.

Interestingly, it was also found that plasma levels of urotensin II in healthy persons are significantly increased by smoking.

## Claims

1. Method of determining the biologically active urotensin II in a sample of a body fluid, comprising contacting the sample with polyclonal and/or monoclonal antibodies produced by immunisation with a peptide having the sequence
ETPDCFWKYC-βAla-C SEQ ID NO: 2
with a disulfide bridge formed between Cys⁵ and Cys¹⁰ and comprising the artificial sequence βAla-Cys, optionally conjugated with a carrier peptide.

2. Method as claimed in claim 1, wherein said antibodies have been purified by their specific binding to the amino acid structure
CFWKYCX SEQ ID NO: 1
wherein C is cystein, F phenylalanine, W tryptophane, K lysine, Y tyrosine, and X is either β-alanine, valine, or isoleucine, wherein the region of CFWKYC is cyclisized by a disulfide bridge.

3. Method as claimed in claim 2, wherein the cyclic peptide is bound to a stationary phase.

4. Method as claimed in any preceding claim 1 to 3, which is a competitive binding assay.

5. Immunoassay as defined in claim 4 for diagnosis of cardiovascular diseases, heptarenal syndrome, cirrhosis, pathological conditions involving haemodynamic alterations, pathological conditions involving vasoconstrictive substances.

6. Immunoassay as defined in claim 4 for diagnosis of neurodegenerative diseases or traumatism of the spinal cord.

## Patentansprüche

1. Verfahren zur Bestimmung des biologisch aktiven Urotensins II in einer Probe einer Körperflüssigkeit, umfassend das Zusammenbringen der Probe mit polyklonalen und/oder monoklonalen Antikörpern, die hergestellt sind durch Immunisierung mit einem Peptid der Sequenz
ETPDCFWKYC-βAla-C SEQ ID Nr: 2,
welches eine Disulfidbrücke zwischen Cys⁵ und Cys¹⁰ hat und das die künstliche Sequenz βAla-Cys umfasst und das gegebenenfalls gekoppelt ist mit einem Trägerpeptid.

2. Verfahren nach Anspruch 1, wobei die Antikörper gereinigt sind durch eine spezifische Bindung an die Aminosäurestruktur
CFWKYCX SEQ ID Nr: 1
wobei C Cystein, F Phenylalanin, W Tryptophan, K Lysin, Y Tyrosin und X β-Alanin, Valin oder Isoleucin ist und die CFWKYC-Region über eine Disulfidbrücke zyklisiert ist.

3. Verfahren nach Anspruch 2, wobei das zyklische Peptid an eine stationäre Phase gebunden ist.

4. Verfahren nach einem der Ansprüche 1 bis 3, bei dem es sich um einen kompetitiven Bindungstest handelt.

5. Immuntest gemäß der Definition in Anspruch 4 für die Diagnose von kardiovaskulären Erkrankungen, hepatorenalem Syndrom, Zirrhose, pathologischen Zuständen, die hämodynamische Veränderungen einschließen, und pathologischen Zuständen, die vasokonstriktive Substanzen einschließen.

6. Immuntest gemäß der Definition in Anspruch 4 für die Diagnose von neurodegenerativen Erkrankungen oder Traumata des Rückenmarks.

## Revendications

1. Procédé pour la détermination d'urotensine II biologiquement active dans un échantillon de fluides corporels, comprenant la mise en contact de l'échantillon avec des anticorps polyclonaux et / ou monoclonaux produits par immunisation avec un peptide ayant la séquence suivante :
ETPDCFWKYC - βAla - C SEQ ID NO : 2
avec un pont disulfure formé entre Cys⁵ et Cys¹⁰ et comprenant la séquence artificielle βAla - Cys, conjuguée de façon optionnelle avec un peptide vecteur.

2. Procédé selon la revendication 1, où lesdits anticorps ont été purifiés grâce à leurs liaisons spécifique avec la structure d'acide aminé suivante :
CFWKYCX SEQ ID NO : 2
où C est cystéine, F phénylalanine, W tryptophane, K lysine, Y tyrosine et X soit β-alanine, valine ou isoleucine, où la région de CFWKYC est cyclisée par un pont disulfure.

3. Procédé selon la revendication 2, où le peptide cyclique est lié à une phase stationnaire.

4. Procédé selon n'importe laquelle des revendications précédentes 1 à 3, qui est un test de liaison compétitive.

5. Immunoessai selon la revendication 4 pour le diagnostic des maladies cardiovasculaires, du syndrome heptarénal, de la cirrhose, des états pathologiques impliquant des altérations haemodynamiques, des états pathologiques impliquant des substances vasoconstrictives.

6. Immunoessai selon la revendication 4 pour le diagnostic de maladies neurogénératives ou des traumatismes de la moelle épinière.


### SEQUENCE LISTING

<110> Immundiagnostik AG
<120> Method of Determining Urotensin II in Body Fluids and Diagnosis of cardiovascular Diseases
<130> P1195EP00/UMB
<140> EP 1241 479 (01106463.1)
   <141> 2003-03-23
<150> ep 01105418
   <151> 2001-03-12
<160> 5
<170> PatentIn version 3.3
<210> 1
   <211> 7
   <212> PRT
   <213> Homo sapiens
<220>
   <221> DISULFID
   <222> (1)..(6)
<220>
   <221> MISC_FEATURE
   <222> (7)..(7)
<400> 1
   Cys Phe Trp Lys Tyr Cys Xaa
   1 5
<210> 2
   <211> 12
   <212> PRT
   <213> Homo sapiens
<220>
   <221> DISULFID
   <222> (5)..(10)
<220>
   <221> MIS_FEATURE
   <222> (11)..(11)
   **<223>** β-alanine
<400> 2
   Glu Thr Pro Asp Cys Phe Trp Lys Tyr Cys Ala Cys
   1 5 10
<210> 3
   <211> 26
   <212> DNA
   <213> Homo sapiens
<400> 3
   tgaaggtcgg agtcaacgga tttggt 26
<210> 4
   <211> 24
   <212> DNA
   <213> Homo sapiens
<400> 4
   catgtgggcc atgaggtcca ccac 24
<210> 5
   <211> 30
   <212> DNA
   <213> Homo sapiens
<400> 5
   ccatacaaga aacgtgagac tcctgattgc 30